# EUROPEAN PATENT APPLICATION

(11) **EP 4 285 961 A1**
(43) Date of publication of application: **06.12.2023**
(21) Application number: 22176649.6
(22) Date of filing: 01.06.2022
(51) Int. Cl.: A61M 5/24, A61M 5/32

(54) **DRUG DELIVERY DEVICE WITH END-OF-DOSE DETECTION**

(71) Applicant: Becton, Dickinson and Company, Franklin Lakes, NJ 07417-1880 (US)
(72) Inventor: CHANSAVANG, Albert, 38000 Grenoble (FR)
(74) Representative: Germain Maureau

(57) **Abstract**

Provided herein is a sensor assembly for a drug delivery device, including a housing defining an interior, the housing having at least one opening therein and being coupleable to a drug delivery device, a switch received at least partially within the interior and having an open configuration and a closed configuration, the switch configured to interact with a cooperating surface on the drug delivery device, and a communication device in electrical communication with the switch.

## Description

### BACKGROUND OF THE INVENTION

### Field of the Invention

The present invention relates generally to drug delivery devices and components therefor, and in particular, to components allowing for detection and communication of the injection status of drug delivery devices.

### Description of Related Art

Drug delivery devices have traditionally lacked features that would allow a healthcare provider to confirm appropriate administration of medicaments. Ensuring timely, appropriate administration of some medicaments can be critical, particularly, for example, in the instance of the evaluation of new drugs in clinical trials, where accurate information is essential.

While some devices provide the ability to communicate certain types of information, for example as described in International Patent Application Publication Nos. WO 2016/087512, WO 2017/070391, WO 2018/111969, WO 2018/213837, and WO 2021/094797, and in U.S. Patent Application Publication Nos. 2019/0083708, 2019/0321555, and 2019/0344019, a need exists in the art for a cost-effective device that accurately detects injection status, and can communicate this drug delivery information to appropriate stakeholders.

### SUMMARY OF THE INVENTION

Provided herein is a sensor assembly for a drug delivery device, including a housing defining an interior, the housing having at least one opening therein and being coupleable to a drug delivery device, a switch received at least partially within the interior and having an open configuration and a closed configuration, the switch configured to interact with a cooperating surface on the drug delivery device, and a communication device in electrical communication with the switch.

Also provided herein is a drug delivery device, including a syringe with a barrel having a proximal end, a distal end having a cooperating surface, a needle arranged at the distal end, and a sidewall arranged between the proximal end and the distal end defining an interior configured to hold a composition, a plunger rod received at least partially within the interior of the syringe, the plunger rod comprising a proximal end, a distal end, and a stopper arranged at the distal end, a displaceable needle shield, a spring having a compressed configuration and an expanded configuration, the spring configured to displace the needle shield from a first, proximal position in which the displaceable needle shield does not surround the needle of the syringe to a second, distal position in which the displaceable needle shield at least partially surrounds the needle of the syringe, and a sensor assembly for a drug delivery device, including a housing defining an interior, the housing having at least one opening therein and being coupleable to a drug delivery device, a switch received at least partially within the interior and having an open configuration and a closed configuration, the switch configured to interact with a cooperating surface on the drug delivery device, and a communication device in electrical communication with the switch, wherein the switch of the sensor assembly is configured to interact with the cooperating surface.

### BRIEF DESCRIPTION OF THE DRAWINGS

**FIG. 1** is a perspective view of a non-limiting embodiment of a drug delivery device as described herein;
**FIG. 2** is a perspective view of a non-limiting embodiment of a drug delivery device as described herein;
**FIGS. 3A and 3B** are perspective views of a non-limiting embodiment of a sensor assembly as described herein;
**FIG. 4** is an exploded view of a non-limiting embodiment of a sensor assembly as described herein;
**FIGS. 5A and 5B** are perspective views of a non-limiting embodiment of a sensor assembly as described herein;
**FIGS. 6A** **and** **6B** show cross-section and perspective views of a non-limiting embodiment of a drug delivery device in a pre-use configuration as described herein;
**FIGS. 7A** **and** **7B** show cross-section and perspective views of a non-limiting embodiment of a drug delivery device in a post-use configuration as described herein;
**FIG. 8** is a diagram of a non-limiting embodiment of an environment in which systems, devices, and/or methods described herein may be implemented; and
**FIG. 9** is a diagram of a non-limiting embodiment of components of one or more devices of **FIG. 8****.**

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

As used herein, the terms "communication" and "communicate" refer to the receipt or transfer of one or more signals, messages, commands, or other type of data. For one unit (e.g., any device, system, or component thereof) to be in communication with another unit means that the one unit is able to directly or indirectly receive data from and/or transmit data to the other unit. This may refer to a direct or indirect connection that is wired and/or wireless in nature. Additionally, two units may be in communication with each other even though the data transmitted may be modified, processed, relayed, and/or routed between the first and second unit. For example, a first unit may be in communication with a second unit even though the first unit passively receives data and does not actively transmit data to the second unit. As another example, a first unit may be in communication with a second unit if an intermediary unit processes data from one unit and transmits processed data to the second unit. It will be appreciated that numerous other arrangements are possible. Any known electronic communication protocols and/or algorithms can be used such as, for example, TCP/IP (including HTTP and other protocols), WLAN (including 802.11a/b/g/n and other radio frequency-based protocols and methods), analog transmissions, Global System for Mobile Communications (GSM), 3G/4G/LTE, BLUETOOTH, ZigBee, EnOcean, TransferJet, Wireless USB, and the like known to those of skill in the art. In some non-limiting embodiments, a message may refer to a network packet (e.g., a data packet and/or the like) that includes data.

As used herein, the term "computing device" may refer to one or more electronic devices configured to process data. A computing device may, in some examples, include the necessary components to receive, process, and output data, such as a processor, a display, a memory, an input device, a network interface, and/or the like. A computing device may be a mobile device. As an example, a mobile device may include a cellular phone (e.g., a smartphone or standard cellular phone), a portable computer, a wearable device (e.g., watches, glasses, lenses, clothing, and/or the like), a personal digital assistant (PDA), and/or other like devices. A computing device may also be a desktop computer or other form of non-mobile computer.

As used herein, "electrical communication," refers to a state of communication, for example through one or more leads and/or antennas, by which one or more devices, such as an NFC device, BLUETOOTH DEVICE, or the like, can detect and/or measure current through a circuit to thereby determine the orientation of a switch (e.g., whether a circuit of which the switch is a part is open or closed).

Provided herein are drug delivery devices, systems including drug delivery devices, and methods using such systems and devices. Drug delivery devices described herein, as well as systems and methods including the same, include a sensor assembly including a communication device to allow for monitoring of status of the drug delivery device, and communication of that status.

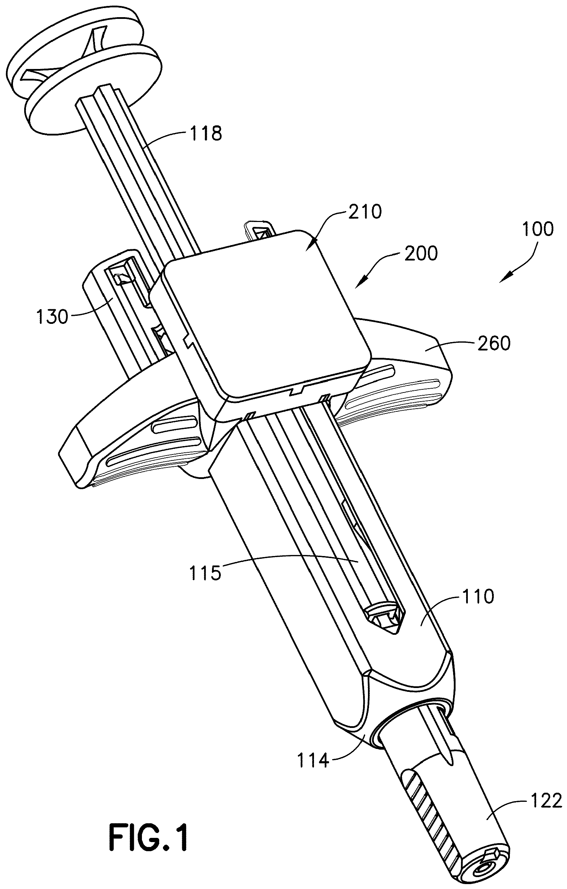

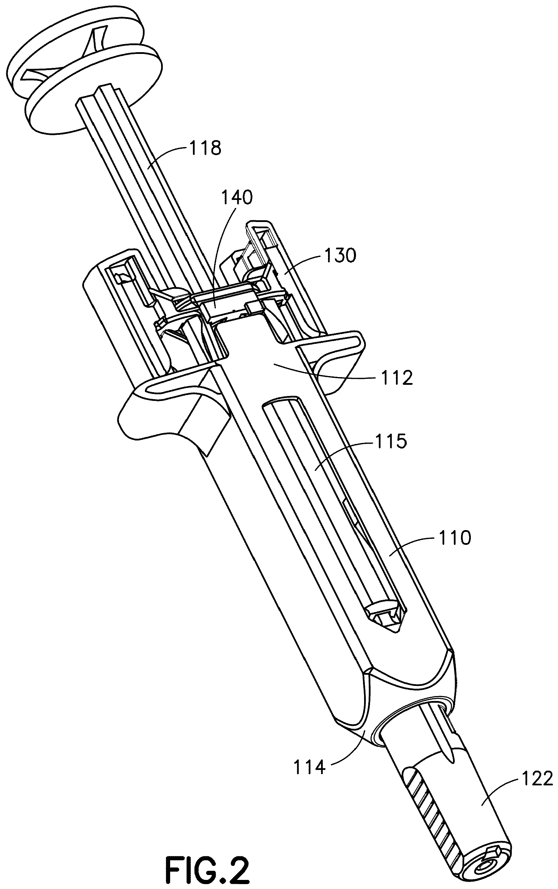

Turning to the figures, shown are a non-limiting embodiments of a drug delivery device 100. Drug delivery device 100, while shown as a device similar to that disclosed in U.S. Patent No. 10,702,663, which is incorporated by reference herein in its entirety, may be any drug delivery device, such as a manual syringe, an autoinjector, a pen injector, or a wearable pump (e.g., an on-body injector). In the non-limiting embodiments, drug delivery device 100 includes a syringe 115 having a proximal end, a distal end, and a sidewall therebetween defining an interior configured to hold a composition (e.g., a therapeutic composition), and a needle 116 arranged at the distal end in fluid communication with the interior. Drug delivery device 100 may also include a plunger rod 118 having a proximal end (optionally including a thumbpad), a distal end, and a stopper 119 arranged at distal end thereof. Drug delivery device 100 may include a displaceable needle shield 110 having a proximal end 112, a distal end 114, and a spring 120 configured to displace needle shield 110 from a first, proximal position in which needle shield 110 does not surround needle 116 to a second, distal position in which needle shield 110 surrounds needle 116. In the non-limiting embodiment of FIGS. 1 and 2, drug delivery device 100 further includes a clip 130, which may be configured to prevent removal of plunger rod 118 from drug delivery device 100. In non-limiting embodiments, drug delivery device 100 further includes a rigid needle shield (RNS) 122 arranged around needle 116. Suitable RNSs, which may include an elastomeric internal component surrounded by a rigid component, are known to those of skill in the art.

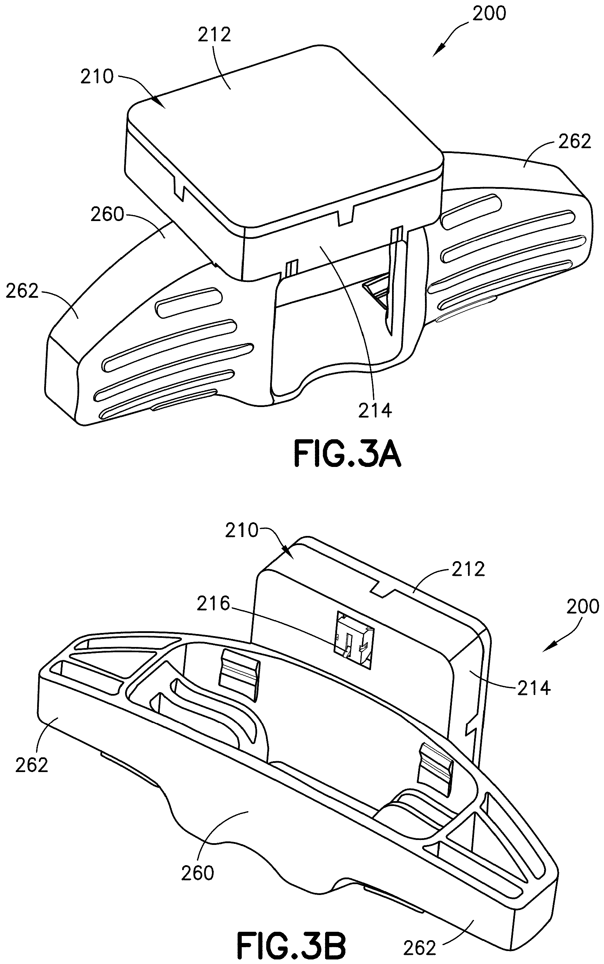

With reference to FIGS. 1, 3A, and 3B, drug delivery device 100 includes a sensor assembly 200, which may be coupled, optionally releasably, to needle shield 110. While sensor assembly 200 is shown as having a parallelogram shape, those of skill in the art will appreciate that any size and/or shape may be used, so long as the sensor assembly can function as described herein. Sensor assembly 200 may include a housing 210, which may be a multi-component housing and may include an opening therethrough to allow one or more parts of a switch (as described below) to pass therethrough or have access to environment outside of housing 210. A multi-component housing may include a first housing part 212 and a second housing part 214, and the housing parts may be releasably or permanently coupled to one another. In non-limiting embodiments, sensor assembly 200 may further include finger flange 260, which may have one or more engagement surfaces 262 configured to allow a user to grip drug delivery device 100 for use thereof. Sensor assembly 200 further includes a switch 216 configured to change state when drug delivery device transitions from a pre-use to a post-use configuration. Any suitable switch 216 may be used, for example mechanical and optical switches. In non-limiting embodiments, switch 216 is a mechanical switch, configured to interact with a cooperating surface 140 on drug delivery device 100. For example, with regard to a mechanical switch, the switch 216 may be in a first orientation when drug delivery device 100 is in a pre-use configuration, for example a mechanical switch may interact with cooperating surface 140. As drug delivery device 100 transitions to a post-use configuration and needle shield 110 moves to a second, distal position, the mechanical switch may fall out of interaction with cooperating surface 140. In non-limiting embodiments, a mechanical switch may rotate as needle shield 110, and sensor assembly 200, move distally. In non-limiting embodiments, once switch 216 is rotated, it cannot be rotated back to its initial position, meaning sensor assembly is a one-time use device. In non-limiting embodiments, switch 216 can be rotated back to its initial position, and sensor assembly 200 can be reused. With regard to an optical switch, a change in distance between the optical switch and the cooperating surface 140, and between the optical switch and another portion of drug delivery device 100, may indicate that drug delivery device 100 has transitioned to a post-use configuration.

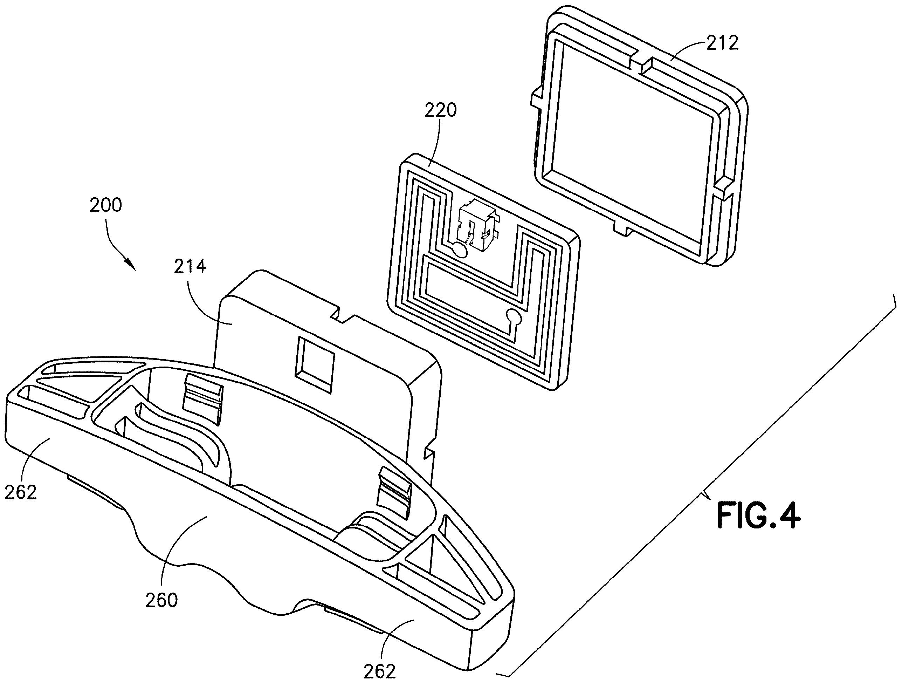

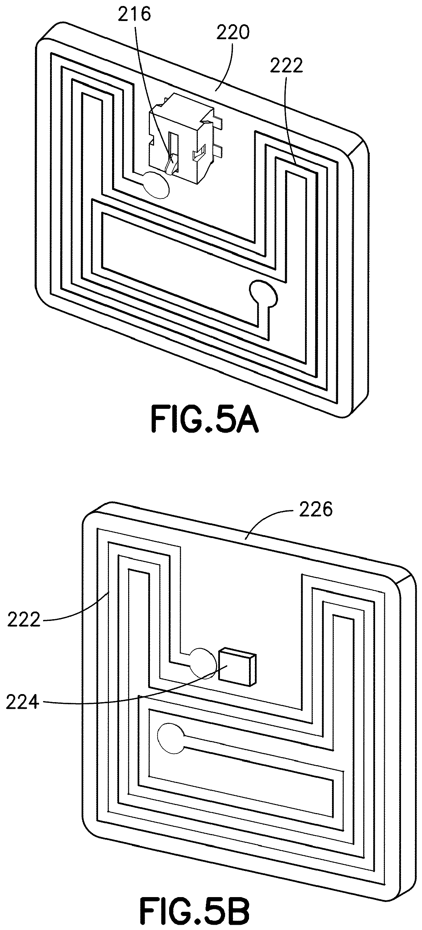

Turning to FIGS. 4-5B, shown are components of sensor assembly 200. As noted, sensor assembly 200 may include one or more housing portions, e.g., housing first part 212 and housing second part 214, and switch 216. Switch 216, as well as communication device 224, including antenna 222, may be provided on a circuit board 220, such as a printed circuit board (PCB) received within housing 210. In non-limiting embodiments, communication device 224 is a short-range communication device, such as a near-field communication (NFC) device. Suitable NFC devices and components therefor are known in the art and are commercially available, for example from NXP Semiconductors (Eindhoven, The Netherlands) and Identiv (Santa Anna, CA). Suitable short-range communication devices may include a chip/tag (optionally including a processor and or memory), for example for storage of data and/or instructions, and an antenna for communication with NFC-enabled devices, such as computing devices described herein. In non-limiting embodiments, sensor assembly 200 is unpowered (e.g., does not include an internal power source, such as a battery). In non-limiting embodiments, sensor assembly 200 is powered by an external source, for example an NFC-enabled computing device as described herein. As NFC technology allows for powering of the short-range communication device wirelessly, in non-limiting embodiments, data may be collected and transmitted without the need for any internal power source, reducing the cost of sensor assembly 200 and allow for reuse without the need to replace a power source.

In non-limiting embodiments, communication device 224 is a BLUETOOTH device, such as a BLUETOOTH low energy device (BLE device), and sensor assembly includes a power source. Those of skill will appreciate that a BLE device may make use of a smaller antenna 222, and thus that sensor assembly housing 210 need not be increased in size to accommodate an internal power source.

In non-limiting embodiments, no matter the communication device 224 that is used, sensor assembly 200 further includes a second communication device, such as an RFID device.

In non-limiting embodiments, sensor assembly 200, with or without finger flange 260, may be reversibly coupled to drug delivery device 100. For example, sensor assembly 200 and/or finger flange 260 may include one or more features to reversible couple with drug delivery device 100 through a snap fit, a friction fit, or other reversible coupling. In non-limiting embodiments, sensor assembly 200 and/or finger flange 260 may include a magnet, to magnetically couple to drug delivery device 100. In non-limiting embodiments, by virtue of being removably coupleable to drug delivery device 100, sensor assembly 200 is reusable.

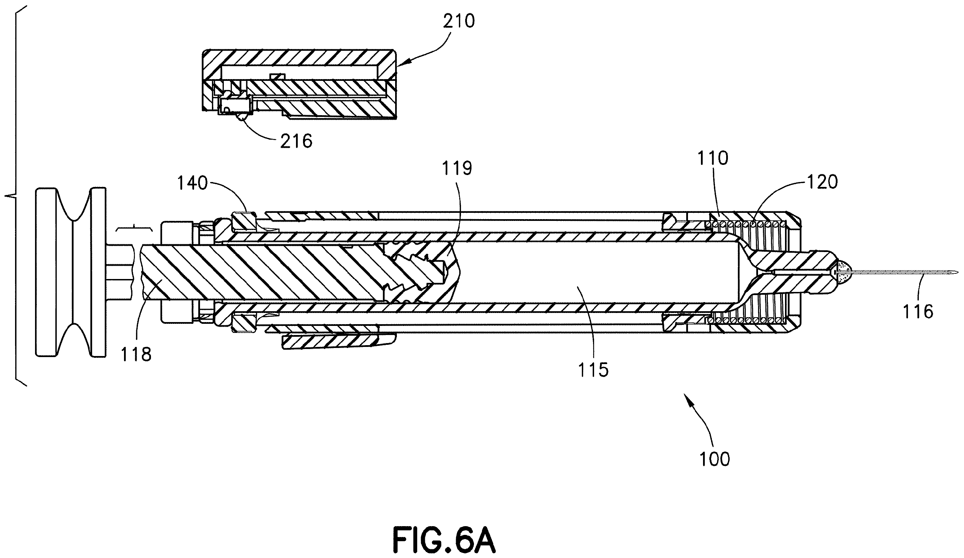

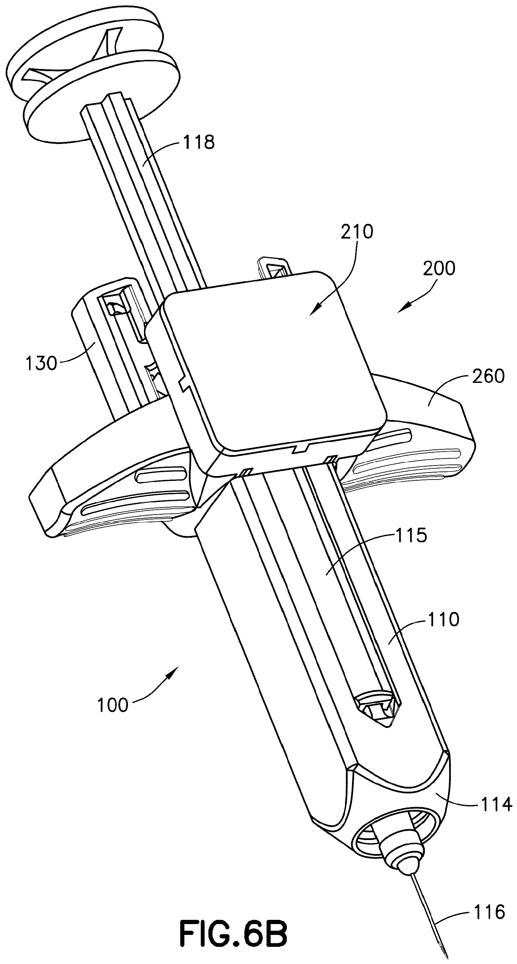

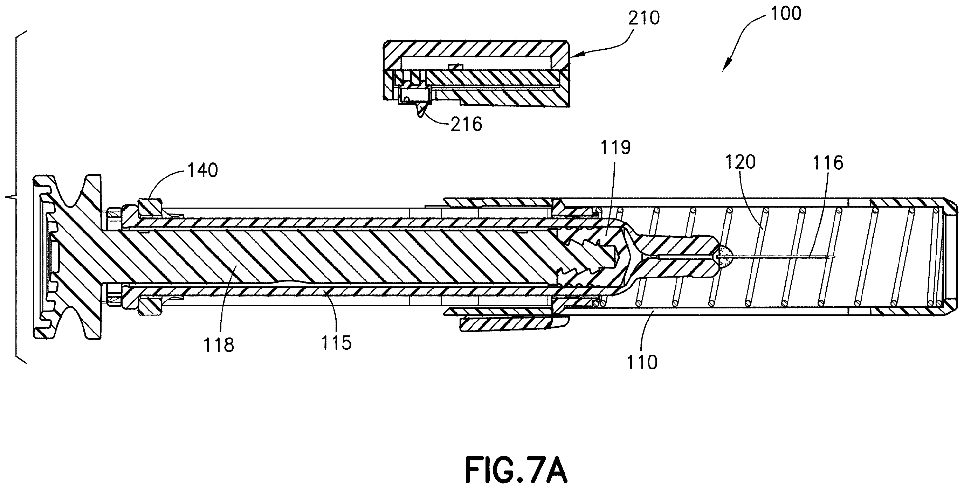

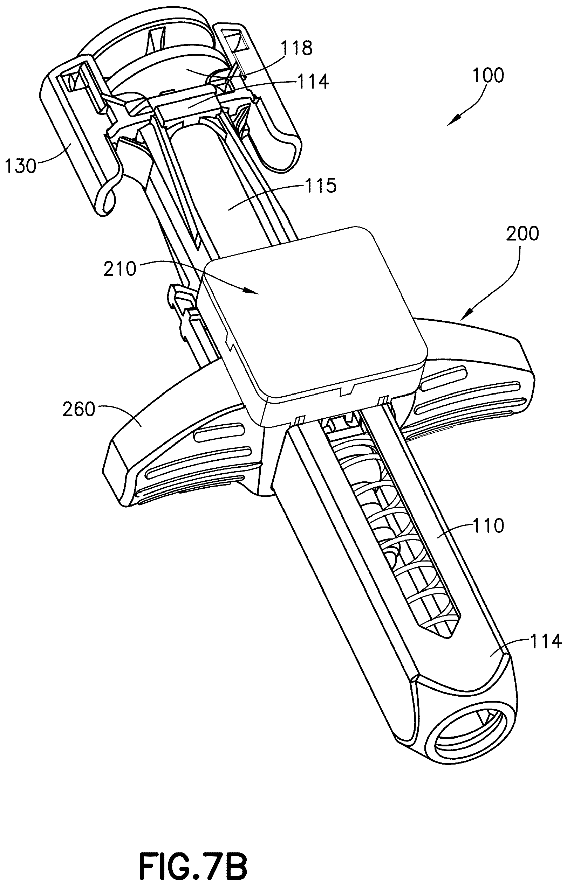

Turning to FIGS. 6A-7B, shown is a non-limiting embodiment of drug delivery device 100 in both pre-use (FIGS. 6A and 6B) and post-use (FIGS. 7A and 7B) configurations. Drug delivery device 100 includes components as described above, including syringe 115, cooperating surface 140, needle shield 110, and sensor assembly 200. As shown in FIG. 6A, in a pre-use position, a non-limiting embodiment of switch 216 is a mechanical switch having a first, pre-use orientation, in which switch 216 interacts with cooperating surface 140. As drug delivery device 100 transitions to a post-use configuration, needle shield 110 moves distally under the influence of spring 120, as spring 120 transitions from a compressed (pre-use) to an expanded (post-use) configuration, shown in FIG. 7A. As this occurs, switch 216 transitions to a second, post-use orientation (for example by rotating as switch 216 is acted upon by cooperating surface 140). In non-limiting embodiments, because switch 216 is only transitioned to a post-use configuration once needle shield 110 is in the process of transitioning to its second, distal position, opening or closing of switch 216 (as described in greater detail below) can reliably be utilized as a proxy for end of drug delivery. In non-limiting embodiments, an advantage of the arrangement of switch 216 and interaction of switch 216 and cooperating surface 140 allows for accurate determination of use status of drug delivery device 100 without any risk of interfering with any aspect of drug delivery device 100 function, and thus drug delivery.

Switch 216 may be part of an electrical circuit, which may include communication device 224 and/or a processor or other component on circuit board 220. Switch 216 may be a normally open (NO) or normally closed (NC) switch. For example, in an NO switch, switch 216 may, in the first, pre-use orientation, be configured such that an electrical circuit is open (e.g., current cannot flow therethrough). Then, as switch 216 transitions to second, post-use orientation, the electrical circuit closes, such that current can flow therethrough. As communication device 224 and/or a processor associated with sensor assembly may be in electrical communication with such a circuit, one or both may detect current and/or determine a use status of drug delivery device 100 based on detected current. Those of skill in the art will appreciate that for an NC switch, the opposite of the foregoing would be true.

Communication device 224, for example an NFC device, may, once powered by an external, NFC-enabled device (such as a user computing device, e.g., a mobile device, as described herein, being brought into proximity with sensor assembly 200), be configured to detect current, and, optionally, determine whether the electrical circuit, including switch 216, is open or closed, and can record current data and/or use status (e.g., a processor associated with sensor assembly 200 may determine, based on current data, whether drug delivery device is in a pre- or post-use configuration) in memory and/or transmit the current data and/or determined use status to a user computing device, such as a mobile device. Those of skill will appreciate that an alternative embodiment, in which communication device 224 is a BLE device, powered by a power source within sensor housing 210, may similarly use energy supplied by power source to detect current, and, optionally, determine whether the electrical circuit, including switch 216, is open or closed, and can record current data and/or use status in memory and/or transmit the current data and/or use status to a user computing device, such as a mobile device.

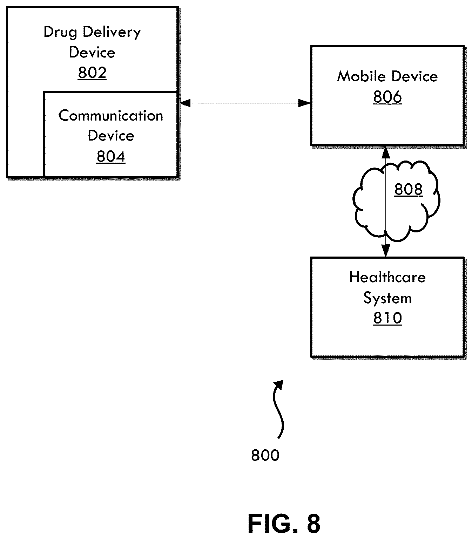

Referring now to FIG. 8, shown is a diagram of an example environment in which devices, systems, and/or methods, described herein, may be implemented. As shown in FIG. 8, the environment can include drug delivery device 802 including communication device 804, user device 806, healthcare system 810, and/or communication network 808. Drug delivery device 802, user device 806, and healthcare system 810 may interconnect (e.g., establish a connection to communicate) via wired connections, wireless connections, or a combination of wired and wireless connections.

Drug delivery device 802, which can be a syringe, autoinjector, wearable injector, and/or pump as described herein, can include, as described above, a sensor assembly including communication device 804 and a switch. As described above, drug delivery device 802 can be configured to communicate with a user device 806.

User device 806 can be a computing device as described herein, in some non-limiting embodiments, a smartphone. User device 806 can be programmed or configured to communicate, for example through communication network 808, with a healthcare system 810, for example through a mobile application executable on user device 806.

Communication network 808 may include one or more wired and/or wireless networks. For example, communication network 808 may include a BLUETOOTH connection (e.g., between drug delivery device 802 and user device 806), a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN) and/or the like), a private network, an ad hoc network, an intranet, the Internet, a fiber optic-based network, a cloud computing network, and/or the like, and/or a combination of some or all of these or other types of networks.

Healthcare system 810 may include a server, a group of servers, and/or other like devices. More than one healthcare system 810 can be provided, for example, a system associated with a device manufacturer, a system associated with a pharmaceutical manufacturer, a system associated with a healthcare provider, a system associated with a government agency, and/or a system associated with a study sponsor, for example a sponsor of a clinical trial.

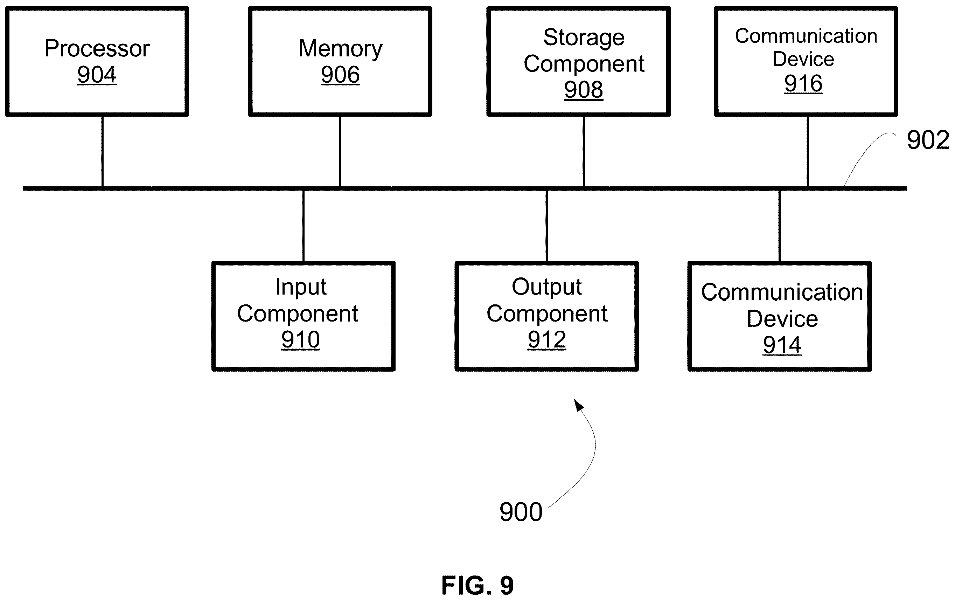

Referring now to FIG. 9, shown is a diagram of example components of an exemplary computing device 900, in an exemplary system, useful for methods described herein. Such a computing device 900 may correspond to a component within the drug delivery device as described herein, a user device as described herein, and/or a healthcare system as described herein. As shown in FIG. 9, a computing device 900 may include bus 902, processor 904, memory 906, storage component 908, input component 910, output component 912, and/or communication interface 914.

Bus 902 may include a component that permits communication among the components of a computing device 900. In some non-limiting embodiments, processor 904 may be implemented in hardware, software, or a combination of hardware and software. For example, processor 904 may include a processor (e.g., a central processing unit (CPU), a graphics processing unit (GPU), an accelerated processing unit (APU), and/or the like), a microprocessor, a digital signal processor (DSP), and/or any processing component (e.g., a field-programmable gate array (FPGA), an application-specific integrated circuit (ASIC), and/or the like) that can be programmed to perform a function. Memory 906 may include random access memory (RAM), read-only memory (ROM), and/or another type of dynamic or static storage memory (e.g., flash memory, magnetic memory, optical memory, and/or the like) that stores information and/or instructions for use by processor 904.

Storage component 908 may store information and/or software related to the operation and use of computing device 900. For example, storage component 908 may include a hard disk (e.g., a magnetic disk, an optical disk, a magneto-optic disk, a solid state disk, and/or the like), a compact disc (CD), a digital versatile disc (DVD), a floppy disk, a cartridge, a magnetic tape, and/or another type of computer-readable medium, along with a corresponding drive.

Input component 910 may include a component that permits computing device 900 to receive information, such as via user input (e.g., a touch screen display, a keyboard, a keypad, a mouse, a button, a switch, a microphone, and/or the like). Additionally or alternatively, input component 910 may include a sensor for sensing information (e.g., a global positioning system (GPS) component, an accelerometer, a gyroscope, an actuator, and/or the like). Output component 912 may include a component that provides output information from a computing device (e.g., a display, a speaker, one or more light-emitting diodes (LEDs), and/or the like).

Communication interface 914 may include a transceiver-like component (e.g., a transceiver, a separate receiver, and transmitter, etc.) that enables device to communicate with other devices, such as via a wired connection, a wireless connection, or a combination of wired and wireless connections. Communication interface 914 may permit computing device 900 to transmit and/or receive information from another device. For example, communication interface 914 may include an Ethernet interface, an optical interface, a coaxial interface, an infrared interface, a radio frequency (RF) interface, a universal serial bus (USB) interface, a Wi-Fi^{®} interface, a cellular network interface, BLUETOOTH interface, and/or the like. In non-limiting embodiments, communication interface 914 operates through one or both of near-field communication and RFID. Suitable communication protocols and methods for securing communications between communication interface 914 and a communication interface of another device, such as a computing device (e.g., desktop computer, laptop computer, smartphone, smart watch, PDA, tablet, etc.,) can include encryption, e.g., using a secure socket layer (SSL) (e.g., by using public/private key pairs as are known in the art). Additional security protocols are disclosed in, for example, U.S. Patent Nos. 9,445,264 and 9,463,325, the contents of which are hereby incorporated by reference in their entirety.

A computing device may perform one or more processes described herein. A computing device may perform these processes based on processor 904 executing software instructions stored by a computer-readable medium, such as memory 906 and/or storage component 908, and/or being instructed by a separate computing device. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 906 and/or storage component 908 from another computer-readable medium or from another device via communication interface 914. When executed, software instructions stored in memory 906 and/or storage component 908 may cause processor 904 to perform one or more processes described herein. Additionally or alternatively, hardwired circuitry may be used in place of or in combination with software instructions to perform one or more processes described herein. Thus, embodiments described herein are not limited to any specific combination of hardware circuitry and software.

With reference once again to FIG. 8, in non-limiting embodiments, an exemplary system 800 may include a drug delivery device 802 including communication device 804 as described previously. The drug delivery device 802, through communication device 804, may thus be in communication with a user's computing device 806, such as a smartphone, having its own associated communication interface and processor, as well as memory, storage component, bus, input component, and/or output component.

In non-limiting embodiments, communication device 804 is in one-way communication with a user's computing device 806, e.g., the short-range communication device 804 can only transmit data to the user device 806, and cannot receive data from the user device 806.

As described above, in non-limiting embodiments, upon being placed near drug delivery device 802, user's computing device 806, by virtue of being enabled for NFC, can deliver power to sensor assembly including communication device 804, which can, based on communication device 804 being in electrical communication with a circuit including a switch as described herein, determine current data and/or determined use status of the drug delivery device 802, which may be transmitted to user's computing device 806, optionally with one or more identifiers (e.g., a device identifier, drug identifier, lot identifier, and/or an identifier associated with communication device 804), to user device 806. In non-limiting embodiments, current data, use status, and/or one or more identifiers are encrypted before being transmitted to user device 806. In addition, other sensors can be provided that are in communication with short-range communication device 804 (or any other included communication device, such as an RFID device) and allow for transmission of, for example, GPS data, gyroscope data, temperature data, humidity data, light exposure data, and the like, which may be relevant to any composition that is to be held within drug delivery device 802.

In non-limiting embodiments, user device 806 and/or healthcare system 810, based on software installed thereon, may compare current data and/or use status received from drug delivery device 802 to one or more pre-determined current values. Such pre-determined current values may be stored in memory of user device 806 and/or healthcare system 810, and may include predicted or actual current values for various stages of drug delivery. For example, pre-determined current values may include current measured when the drug delivery device is in a pre-use configuration (e.g., switch is opened or closed) and a post-use configuration (e.g., switch is closed or open). Results of the comparison(s), as well as received current data and/or one or more identifiers and/or additional data described herein, may be stored on one or both of user device 806 and/or healthcare system 810, and transmitted therebetween via network 808. In non-limiting embodiments, if any current data and/or use status falls outside of a predetermined range, a message can be generated by or sent to user device 806. In addition, data may be stored by user device 806 and/or healthcare system 810, such that if a particular sensor assembly (e.g., communication device 804) is believed to have not been used previously, but data is detected and/or stored that is indicative of current and/or use status in a post-use configuration, an alert may be sent to the user device 806 that the drug delivery device 802 may have been previously used or may have been tampered with.

In non-limiting embodiments, healthcare system 810 may be in communication with one or more additional healthcare systems 810. For example, one healthcare system may be maintained by the device manufacturer, and may be in communication with a healthcare system maintained by a study sponsor, pharmaceutical manufacturer, or the like. In non-limiting embodiments, current data (e.g., first current data, second current data, etc. and/or any comparisons between the measured current data and predetermined current levels), together with one or more identifiers (e.g., a device identifier and/or an identifier associated with the short-range communication device 804), may be transmitted from device manufacturer system to study sponsor or pharmaceutical manufacturer system, where one or more databases may store data associating the one or more identifiers (e.g., a device identifier and/or an identifier associated with the short-range communication device 804) with a patient identifier, allowing for association of any collected data with the patient.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A sensor assembly for a drug delivery device, comprising:
a housing defining an interior, the housing having at least one opening therein and being coupleable to a drug delivery device;
a switch received at least partially within the interior and having an open configuration and a closed configuration, the switch configured to interact with a cooperating surface on the drug delivery device; and
a communication device in electrical communication with the switch.

2. The sensor assembly of claim 1, further comprising a finger flange coupleable to the housing.

3. The sensor assembly of claim 1 or claim 2, wherein the switch is a mechanical switch.

4. The sensor assembly of claim 1 or claim 2, wherein the communication device is a short-range communication device.

5. The sensor assembly of claim 4, wherein the short-range communication device is a near-field communication (NFC) device.

6. The sensor assembly of claim 5, wherein the sensor assembly does not include a power source.

7. The sensor assembly of claim 1 or claim 2, wherein the communication device comprises a BLUETOOTH device.

8. The sensor assembly of claim 7, wherein the sensor assembly further comprises a power source at least partially received within the housing.

9. The sensor assembly of claim 1 or claim 2, wherein the switch is a mechanical switch, and wherein the mechanical switch comprises an arm extending at least partially through the opening in the housing.

10. A drug delivery device, comprising:
a syringe comprising a barrel having a proximal end, a distal end having a cooperating surface, a needle arranged at the distal end, and a sidewall arranged between the proximal end and the distal end defining an interior configured to hold a composition;
a plunger rod received at least partially within the interior of the syringe, the plunger rod comprising a proximal end, a distal end, and a stopper arranged at the distal end;
a displaceable needle shield;
a spring having a compressed configuration and an expanded configuration, the spring configured to displace the needle shield from a first, proximal position in which the displaceable needle shield does not surround the needle of the syringe to a second, distal position in which the displaceable needle shield at least partially surrounds the needle of the syringe; and
the sensor assembly of claim 1, coupled to the needle shield, wherein the switch of the sensor assembly is configured to interact with the cooperating surface.

11. The drug delivery device of claim 10, wherein the sensor assembly further comprises a finger flange coupled to the housing.

12. The drug delivery device of claim 10 or claim 11, wherein the sensor assembly is reversibly coupleable to the needle shield.

13. The drug delivery device of claim 10 or claim 11, wherein the switch is a mechanical switch, and wherein, when the needle shield is in the first, proximal position, the mechanical switch interacts with the cooperating surface of the syringe and when the needle shield is in the second, distal position, the mechanical switch does not interact with the cooperating surface.

14. The drug delivery device of claim 13, wherein the switch comprises a rotatable arm, and wherein the arm rotates as the shield moves from the first, proximal position to the second, distal position.

15. The drug delivery device of claim 10 or claim 11, wherein the communication device is an NFC device, and wherein the sensor assembly does not include an internal power source.
